(19) Europäisches Patentamt
European Patent Office
Office européen des brevets

(11) Veröffentlichungsnummer: **0 094 347**
**B1**

# (12) EUROPÄISCHE PATENTSCHRIFT

(45) Veröffentlichungstag der Patentschrift: **10.07.85**

(51) Int. Cl.⁴: **C 07 C 49/82**, C 07 C 49/825, C 07 C 49/83, C 07 C 45/64

(21) Anmeldenummer: **83810184.8**

(22) Anmeldetag: **02.05.83**

## (54) Verfahren zur Herstellung von 1-Hydroxyketonen.

(30) Priorität: **07.05.82 CH 2842/82**

(43) Veröffentlichungstag der Anmeldung: **16.11.83 Patentblatt 83/46**

(45) Bekanntmachung des Hinweises auf die Patenterteilung: **10.07.85 Patentblatt 85/28**

(84) Benannte Vertragsstaaten: **DE GB IT NL**

(56) Entgegenhaltungen:
**DE - A - 2 722 264**
**DE - A - 2 808 459**

(73) Patentinhaber: **CIBA-GEIGY AG, Postfach, CH-4002 Basel (CH)**

(72) Erfinder: **Stegmann, Werner, Dr., Unter der Sonnhalde 9, CH-4410 Liestal (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 094 347 B1

BUNDESDRUCKEREI BERLIN

**Beschreibung**

Die Erfindung betrifft ein neues Verfahren zur Herstellung von 1-Hydroxyketonen.

1-Hydroxyketone sind bekanntlich wirkungsvolle Initiatoren für die Photopolymerisation ungesättigter Verbindungen und photochemische Vernetzer für Polyolefine. Derartige Verwendungen werden beispielsweise in dem EP-Patent 3002 beschrieben.

Nach einem bekannten Verfahren zur Herstellung von 1-Hydroxyketonen werden die entsprechenden $\alpha$-Halogenketone durch direkte Hydrolyse oder durch Hydrolyse über die Stufe der entsprechenden Ameisensäure- oder Essigsäureester in die gewünschten Produkte überführt. Diesbezüglich ist z. B. auf eine Veröffentlichung von D. Mayer »Hydroxy-Ketone und deren Ester« in Houben-Weyl, Bd. VII/2c, Seiten 2171—2243, (1977) hinzuweisen. Es ist bekannt, daß dieses bekannte Verfahren erhebliche Nachteile aufweist. So wird dasselbe nämlich durch eine basenkatalysierte Isomerisierung der $\alpha$-Hydroxy-Ketone erheblich gestört und es fällt ein entsprechend unreines Endprodukt in nur mäßiger Ausbeute an. Als weitere Komplikation bei einer solchen Reaktion wird in obiger Publikation die Möglichkeit von Faworsky-Umlagerungen herausgestellt, die ebenfalls zu einem Isomerengemisch führen, das neben dem erwünschten Hydroxyketon auch die isomere Carbonsäure enthält.

Speziell die Herstellung von Hydroxyalkylphenon ist auch in den deutschen Offenlegungsschriften DE-OS 2 722 264 und DE-OS 2 808 459 vorbeschrieben. Dort wird ein Benzolderivat in Gegenwart einer Lewis-Säure mit einem $\alpha$-Halogencarbonsäurechlorid umgesetzt, und das so erhaltene $\alpha$-Halogenketon wird dann in an sich üblicher Weise zum Hydroxyalkylphenon verseift.

Ein weiteres bekanntes Verfahren zur Herstellung von 1-Hydroxyketonen wird ebenfalls in der oben angeführten Publikation auf Seite 2177 usw. beschrieben. Man hydrolysiert Halogenepoxide in saurem oder aliphatischem Milieu. Auch dieses Verfahren weist den Nachteil auf, daß die Ausbeute gering ist und daß erheblich unreine Endprodukte anfallen.

Es ist auch noch besonders hervorzuheben, daß der erforderliche technische Aufwand aller bekannten Verfahren besonders groß ist.

Das erfindungsgemäße Verfahren weist nun überraschend die Nachteile der bekannten Verfahren zur Herstellung von 1-Hydroxyketonen nicht auf. Es fallen sehr reine Produkte in hoher Ausbeute an. Die technischen Anlagen zur Durchführung des neuen Verfahrens sind einfach und wenig aufwendig.

Gegenstand der Erfindung ist ein Verfahren zur Herstellung von Verbindungen der Formel I

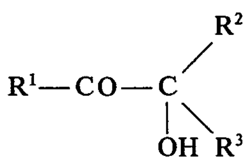

(I)

in der $R^1$ einen Phenylrest, welcher durch geradkettige oder verzweigte $C_1$—$C_4$-Alkylreste substituiert sein kann, bedeutet, $R^2$ und $R^3$ gleich oder verschieden sind, und einen geradkettigen oder verzweigten $C_1$—$C_5$-Alkylrest, einen $C_5$—$C_8$-Cycloalkylrest, welcher durch 1 bis 3 geradkettige oder verzweigte $C_1$—$C_5$-Alkylreste substituiert sein kann, oder zusammen mit dem C-Atom, an das sie gebunden sind, einen $C_5$—$C_8$-Cycloalkylrest, welcher durch 1 bis 3 geradkettige oder verzweigte $C_1$—$C_4$-Alkylreste substituiert sein kann, bedeuten, durch Umsetzung einer Verbindung der Formel II

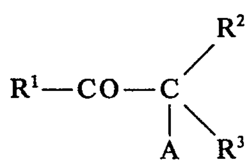

(II)

in der $R^1$, $R^2$ und $R^3$ wie oben definiert sind und A Chlor oder Brom, vorzugsweise Chlor, bedeutet, mit Hydroxidionen erzeugenden Verbindungen in Gegenwart von Wasser oder in Gegenwart von Wasser und einem inerten organischen Lösungsmittel nach dem Phasentransfer-Katalyse-Verfahren, wobei ein Phasentransfer-Katalysator eingesetzt wird und die Verbindung der Formel II in Lösung oder geschmolzen vorliegt.

$R^1$ kann außer Phenyl insbesondere einen o-, m- oder p- Toluyl- oder -Xylylrest bedeuten. $R^2$ und/oder $R^3$ können folgende Alkylreste bedeuten: Zum Beispiel Methyl, iso-Propyl, n-Butyl, sec.-Butyl, tert.-Butyl, tert.-Pentyl. Als Cycloalkylreste können sie beispielsweise Cyclopentyl, Cyclohexyl, Cycloheptyl oder Cyclooctyl bedeuten. Bevorzugt sind Cyclopentyl und Cyclohexyl.

Bilden $R^2$ und $R^3$ zusammen mit dem C-Atom, an das sie gebunden sind, einen Cycloalkylrest, so kann es sich um dieselben bereits genannten Reste handeln.

Bevorzugt werden Verbindungen der Formel I hergestellt, in der $R^1$ einen unsubstituierten oder durch eine oder mehrere $C_1$—$C_4$-Alkylgruppen substituierten Phenylrest, $R^2$ und $R^3$ beide Methyl oder zusammen mit dem C-Atom, an das sie gebunden sind, Cyclohexyl bedeuten, indem man entsprechende Verbindungen der Formel II mit Hydroxidionen erzeugenden Verbindungen umsetzt.

Eine besondere Vorzugsform der Erfindung stellt die Herstellung einer Verbindung aus der Reihe der Formeln

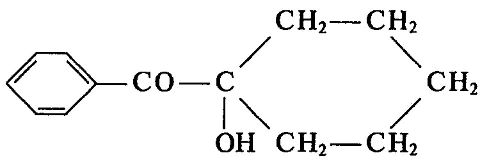

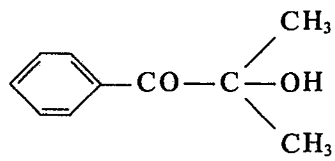

und

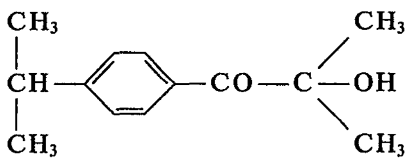

dar, wobei man als entsprechende Verbindung der Formel II 1-Chlorcyclohexyl-phenylketon, 1-Chlorisopropyl-phenylketon oder 1-Chlorisopropyl-4'-isopropylphenylketon einsetzt.

Die erfindungsgemäße Umsetzung erfolgt vorzugsweise bei Temperaturen zwischen 0° und 120° C, insbesondere zwischen 60° und 85° C.

Für die Erzielung guter Ergebnisse sind dabei Reaktionszeiten von 2 bis 5 Stunden, vorzugsweise etwa 3 Stunden, erforderlich.

Nach einer Vorzugsform der Erfindung liegt die Verbindung der Formel II geschmolzen in dem Reaktionsgemisch vor und es wird in Abwesenheit von inerten organischen Lösungsmitteln umgesetzt.

Werden inerte organische Lösungsmittel eingesetzt, so handelt es sich dabei beispielsweise um aromatische Kohlenwasserstoffe wie Benzol, Toluol, Xylole, aliphatische oder cycloaliphatische Kohlenwasserstoffe wie Heptan, Hexan, Benzin, ferner Ketone, Ester oder Äther und Glycoläther.

Hydroxidionen erzeugende Verbindungen sind beispielsweise Alkali- oder Erdalkalihydroxide. Die eingesetzte Menge beträgt vorzugsweise 100—150 Gew.-% der theoretischen Menge. Die Verbindungen der Formel II werden vorzugsweise mit KOH oder NaOH umgesetzt.

Es ist vorteilhaft, die Hydroxydionen erzeugenden Verbindungen als wäßrige Lösungen einzusetzen, so daß das im Laufe der Reaktion entstehende Halogenidsalz im Reaktionsmedium in Lösung bleibt.

Als Phasentransfer-Katalysatoren werden bevorzugt Verbindungen der Formel III

$$(R^a)\,(R^b)\,(R^c)\,(R^d)\,N^{\oplus}X^{\ominus} \qquad \text{(III)},$$

in der $R^a$, $R^b$, $R^c$ und $R^d$ gleich oder verschieden sind und geradkettige oder verzweigte Alkylreste, wobei alle 4 Alkylreste zusammen 4 bis 20 C-Atome besitzen, oder Benzyl bedeuten, und $X^{\ominus}$-$HSO_4^{\ominus}$ oder $-Cl^{\ominus}$ darstellen, eingesetzt.

$R^a$, $R^b$, $R^c$ und $R^d$ können folgende Alyklreste bedeuten: Zum Beispiel Methyl, iso-Propyl, n-Butyl, sec.-Butyl, tert.-Butyl, tert.-Pentyl, 2-Äthylhexyl, n-Octyl, 1,1,3,3-Tetramethylbutyl, n-Dodecyl.

Die verwendete Katalysatormenge kann beliebig ausgewählt werden, sie liegt jedoch vorzugsweise bei 0,1—2 Mol-%, bezogen auf 1 Mol der Verbindung der Formel II.

Besonders bevorzugt werden als Katalysatoren die Verbindungen aus folgender Reihe eingesetzt:

$$(n-C_4H_9)_4N\,Cl,\ (n-C_4H_9)(C_2H_5)_3\,NHSO_4 \text{ und } (n-C_4H_9)_4NHSO_4.$$

Nach einer Vorzugsform der Erfindung wird als Phasentransfer-Katalysator eine an ein polymeres Festharz gebundene Verbindung der Formel III verwendet. Als polymere Festharze kommen insbesondere organische Hochpolymere auf Basis Polystyrol in Frage.

Gemäß der Erfindung ist eine ausgeprägt intensive Rührung des Reaktionsgemisches besonders vorteilhaft, woraus sich große Ausbeuten und entsprechende Reinheitsgrade des Endproduktes ergeben. Der Einsatz eines Impellerrührers mit Stromstörer hat sich hier als besonders geeignet erwiesen. Beispiele für Intensivrührer sind Schrägblatt- und Scheibenrührer, Mehrstufen-Impulsgegenstrom-Rührer wie MIG®, Interferenz-Mehrstufen-Impulsgegenstrom-Rührer wie INTERMIG®, Wendelrührer oder Leitstrahlrührer.

Die als Ausgangsprodukte verwendeten Verbindungen der Formel II sind bekannt und ihre Herstellung erfolgt nach bekannten Verfahren. Diesbezüglich ist auf die bereits angeführte Publikation von D. Mayer »Hydroxyketone und deren Ester« in Houben-Weyl, Bd. VII/2c, auf das EP-Patent 3002 und auf das Buch von H. O. House »Modern Synthetic Reactions« (Seite 459 usw.) (Verlag Bejamin 1972) hinzuweisen.

In den folgenden Beispielen wird die Erfindung näher erläutert:

## Beispiel 1

Eine Mischung aus 282 g 1-Chlorcyclohexyl-phenylketon, 156 g Wasser und 156 g Kaliumhydroxid (50%ige Lösung) wird in einem Glasrührergefäß mit Rückflußkühler auf 60—65° C erwärmt, wobei das 1-Chlorcyclohexylphenylketon schmilzt. Innerhalb von 30 Minuten wird eine Lösung von 0,4 g Tetrabutylammoniumhydrogensulfat in 6 g Wasser zudosiert. Die Temperatur des Reaktionsgemisches wird bei 60—75° C gehalten. Nach Abklingen der Reaktion werden weitere 1,7 g Tetrabutylammoniumhydrogensulfat gelöst in 25 g Wasser während 30 Minuten zudosiert; die Temperatur wird dabei bei 65—75° gehalten. Das Reaktionsgemisch wird auf 80° C aufgeheizt und während ca. 3 Stunden bei dieser Temperatur ausgerührt. Es ist dann kein Edukt mehr nachzuweisen (Gehalt an 1-Chlorcyclohexyl-phenylketon ≤ 0,1%, DC-Evidenz). Nach dem Abtrennen der wäßrigen Phase bei 70° C werden zur Produktschmelze 170 g Wasser zugegeben. Die Wasserphase wird durch Zugabe von ca. 0,2 g Essigsäure auf einen pH-Wert von 5,5—7 gestellt. Nach Verrühren wird die wäßrige Phase abgetrennt. Die organische Schmelze wird nochmals mit 170 g Wasser gewaschen. Es werden 100 g Toluol zugegeben und das Wasser durch azeotrope Destillation entfernt. Nach dem Abdestillieren der Restmenge an Toluol bei 110° C/1,6 · $10^3$ Pa, verbleiben ca. 257 g rohes 1-Hydroxycyclohexyl-phenylketon, entsprechend 99% der Theorie. Das Produkt kristallisiert langsam aus der Schmelze aus.
Smp.: 45—49° C.

## Beispiel 2

66,8 g 1-Chlorcyclohexyl-phenylketon und 26,4 g 50%iges Natriumhydroxid werden auf 60—65° C erwärmt. Zur Lösung/Schmelze werden unter raschem Rühren 2 g Tetrabutylammoniumhydrogensulfat, gelöst in 10 g Wasser, gegeben. Die Temperatur des Reaktionsgemisches steigt auf ca. 85° C. Nach dreistündigem Ausrühren bei 80° C ist die Reaktion beendet (Gehalt an 1-Chlorcyclohexylphenylketon in dem Reaktionsgemisch ≤ 0,1%; DC-Evidenz). Das Reaktionsgemisch wird mit 50 ml Toluol und 60 g Wasser während 10 Minuten bei ca. 60° C gerührt und dann die untere wäßrige Phase abgetrennt. Die Toluollösung wird mit 60 g Wasser versetzt und der pH-Wert mit einigen Tropfen verdünnter Salzsäure auf pH = 7 gestellt. Nach dem Abtrennen der Wasserphase wird die toluolische Lösung bei 60° C zweimal mit 50 g Wasser gewaschen. Nach dem Abdestillieren des Toluols bei 90° C/2,67 · $10^3$ Pa verbleiben ca. 60,5 g rohes Produkt = 98,5% der Theorie; das Rohprodukt 1-Hydroxycyclohexyl-phenylketon weist einen gaschromatographisch bestimmten Gehalt von 98,4% auf.

## Beispiel 3

66,8 g 1-Chlorcyclohexyl-phenylketon und 37 g Kaliumhydroxid 50% werden auf 60—65° C erwärmt. Unter raschem Rühren werden 1,7 g Tetrabutylammoniumchlorid gelöst in 10 g Wasser zugegeben, wobei die Temperatur bis auf ca. 100° C ansteigt. Nach zweistündigem Ausrühren bei 90—70° C ist die Reaktion beendet und es wird wie in Beispiel 2 beschrieben aufgearbeitet.
Ausbeute: 58,5 g 1-Hydroxy-cyclohexyl-phenylketon = 95,5% der Theorie;
Gehalt: 98,8%, gaschromatographisch (GC) bestimmt.

## Beispiel 4

Eine Mischung aus 66,8 g 1-Chlorcyclohexyl-phenylketon und 37 g Kaliumhydroxid 50%ige wäßrige Lösung werden durch Aufheizen auf 60—65° C in Lösung/zum schmelzen gebracht. Eine Lösung von 1,73 g Benzyltriäthylammoniumhydrogensulfat in 10 g Wasser wird rasch zugegeben, wobei die Temperatur auf 90° C ansteigt. Nach 3,5stündigem Ausrühren bei 60° C ist die Reaktion beendet. Nach Aufarbeitung wie in Beispiel 2, werden 38,5 g 1-Hydroxy-cyclohexyl-phenylketon (63% der Theorie) erhalten; Gehalt 97,4% (GC).

## Beispiel 5

Eine Mischung aus 66,8 g 1-Chlorcyclohexyl-phenylketon und 38,2 g Kaliumhydroxid 50% wird auf 60—65° C aufgeheizt. 1,1 g Tetramethylammoniumhydrogensulfat gelöst in 10 g Wasser werden auf einmal zugegeben; dabei steigt die Temperatur um ca. 7° C an. Das Reaktionsgemisch wird während drei Stunden bei 60° C gerührt. Nach Zugabe von 10 g Wasser wird 5 Stunden bei 80° C und dann 5 Stunden bei 100° C gerührt, bis der Endpunkt der Reaktion erreicht ist. Aufarbeitung wie in Beispiel 2 liefert ca. 50 g Rohprodukt 1-Hydroxycyclohexyl-phenylketon (81% der Theorie); Gehalt: 97,4% (GC).

### Beispiel 6

Eine Mischung aus 66,8 g 1-Chlorcyclohexyl-phenylketon und 38,2 g Kaliumhydroxid 50% wird auf 60—65° C aufgeheizt. 2,7 g Tetrahexylammoniumhydrogensulfat werden gelöst in 10 g Wasser unter starkem Rühren rasch zugegeben, wobei die Innentemperatur auf 108° C ansteigt. Nach zweistündigem Ausrühren bei 60° C wird das Reaktionsgemisch wie im Beispiel 2 aufgearbeitet.
Ausbeute: 55 g 1-Hydroxy-cyclohexyl-phenylketon = 90% der Theorie;
Gehalt: 91,5% (GC).

### Beispiel 7

Eine Mischung aus 133,6 g 1-Chlorcyclohexyl-phenylketon, 244 ml Heptan, 69,4 g Kaliumhydroxid 50%, 20 g Wasser und 4 g Tetrabutylammoniumhydrogensulfat wird auf 80° C aufgeheizt und während 8 Stunden bei dieser Temperatur gerührt. Nach dieser Zeit liegen weniger als 0,2% 1-Chlorcyclohexyl-phenylketon im Reaktionsgemisch vor. Nach Zugabe von 60 g Wasser wird bei 50—60° C während 10 Minuten gerührt und dann die wäßrige Schicht abgetrennt. Die Heptanlösung wird viermal mit je 60 g Wasser gewaschen, dann abgekühlt und bei 15—20° C unter Zusatz von Impfkristallen kristallisiert. Die Suspension wird auf 0° C bis 3° C abgekühlt und nach einstündigem Rühren abfiltriert. Das Nutschgut wird zweimal mit je 60 ml eiskaltem Heptan gewaschen und bei 30—40° C im Vakuum getrocknet.
Ausbeute an 1-Hydroxycyclohexyl-phenylketon 97,5 g = 79,6% der Theorie. Schmelzpunkt: 46—49° C.

### Beispiel 8

In einem Glasrührergefäß mit Rückflußkühler wird eine Mischung von 160,3 g 1-Bromcyclohexyl-phenylketon, 80,8 g 50%igem Kaliumhydroxid und 51 g Wasser auf 60—65° C aufgeheizt. Zu dieser Lösung werden 0,2 g Tetrabutylammoniumhydrogensulfat, gelöst in 3 g Wasser, dosiert. Das Reaktionsgemisch wird eine Stunde bei 60—70° C gehalten. Zu dieser 70° C warmen Lösung werden weitere 0,8 g Tetrabutylammoniumhydrogensulfat, gelöst in 12 ml Wasser, gegeben. Die Reaktionsmischung wird drei Stunden bei 75—80° C ausgerührt. Es ist dann kein Edukt mehr nachweisbar. (Gehalt an 1-Bromcyclohexyl-phenylketon ≤ 0,1%, DC-Evidenz). Nach dem Abtrennen der wäßrigen Phase bei 60° C werden zur Produktschmelze 51 g Wasser zugegeben und das Gemisch während 10 Minuten bei 60—70° C verrührt. Die Wasserphase wird durch Zugabe von 1 ml Essigsäure 80% auf einen pH-Wert von ca. 7 gestellt. Die Wasserphase wird abgetrennt und die Produktschmelze nochmals mit 51 g Wasser gewaschen. Das so erhaltene Rohprodukt wird mit 185 g Heptan Isomerengemisch und 3,7 g Aktivkohle versetzt und während 30 Minuten bei 45—55° C gerührt. Nach einer Klärfiltration wird die Produktlösung auf 22° C abgekühlt und angeimpft. Die erhaltene Suspension wird auf —5 bis —10° C abgekühlt und das Produkt abfiltriert. Das Nutschgut wird mit insgesamt 155 g 0 bis —10° C kaltem Heptan gewaschen. Nach Trocknung bei 30—40° C im Vakuum werden 80,4 g 1-Hydroxycyclohexyl-phenylketon erhalten, entsprechend 66% der Theorie. Schmelzbereich: 43—48° C.

### Beispiel 9

Eine Mischung aus 135,8 g 4'-Isopropylphenyl-(1-chlorisopropyl)-keton, 80,8 g 50%igem Kaliumhydroxid und 51 g Wasser werden auf 60° C aufgeheizt. Nach dem Zulaufenlassen einer Lösung von 0,2 g Tetrabutylammoniumhydrogensulfat in 3 g Wasser wird 30 Minuten bei 60—70° C ausgerührt. Zu dieser 70° C warmen Lösung werden 0,8 g Tetrabutylammoniumhydrogensulfat, gelöst in 12 g Wasser zulaufen lassen. Das Reaktionsgemisch wird während 5 Stunden bei 75—85° C ausgerührt. Danach wird die Wasserphase bei 80° C abgetrennt und die organische Phase mit einer Mischung von 40 g 50%igem Kaliumhydroxid 51 g Wasser und 1 g Tetrabutylammoniumhydrogensulfat, gelöst in 15 g Wasser, versetzt. Nach einstündigem Rühren bei 70—80° C ist dünnschichtchromatographisch kein Edukt mehr feststellbar (Nachweisgrenze 0,1%). Die wäßrige Phase wird bei 60° C abgetrennt. Nach Zugabe von 51 g Wasser zur verbleibenden organischen Phase wird mit 1 ml Essigsäure 80% der pH-Wert auf ca. 7 gestellt und die wäßrige Phase anschließend abgetrennt. Die organische Phase wird nochmals mit 100 g Toluol versetzt, azeotrop entwässert und das Toluol bei 90° C/1,9 · $10^3$ Pa abdestilliert. Es verbleiben 121 g an 4'-Isopropylphenyl-(1-hydroxyisopropyl)keton, entsprechend 98,6% der Theorie, mit einem Siedepunkt von 164° C/3,7 · $10^3$ Pa.

IR-Spektrum in 2%iger $CCl_4$-Lösung; Absorptionsbanden in $cm^{-1}$ bei:
1655 [ C=O]; 2780, 2955 und 3460 [—OH].

Beispiel 10

155 g 1-Chlorisopropyl-phenylketon, 115 g wäßrige 50%ige Kaliumhydroxidlösung, 73 g Wasser und 0,3 g Tetrabutylammoniumhydrogensulfat gelöst in 4,5 g Wasser werden unter Rühren auf 80°C aufgeheizt. Das Reaktionsgemisch wird 30 Minuten bei 80°C ausgerührt und dann nochmals mit 1,2 g Tetrabutylammoniumhydrogensulfat, gelöst in 17 g Wasser, versetzt. Nach einstündigem Ausrühren bei 70—80°C wird nochmals 50 g Wasser zugegeben und dann die wäßrige Phase abgetrennt. Zur Produktschmelze werden 75 g Wasser gegeben und die Mischung wird während 10 Minuten bei 60—70°C verrührt. Der pH-Wert wird mit ca. 5 g Essigsäure 80% auf einen pH-Wert von 7 eingestellt. Die untere wäßrige Phase wird abgetrennt und das Produkt nochmals mit 73 g Wasser gewaschen. Die Produktschmelze wird mit 5,3 g Aktivkohle und 262 g Heptan versetzt und während 10 Minuten bei 50—60°C verrührt. Nach Filtration der Aktivkohle und Abdestillieren des Heptans bei 80°C/2,6 · $10^3$ Pa am Rotationsverdampfer verbleiben 130 g an 1-Hydroxyisopropyl-phenylketon, entsprechend 93% der Theorie.
Siedepunkt: 134°C/3,3 · $10^3$ Pa.
IR-Spektrum in 2%iger $CCl_4$-Lösung; Absorptionsbanden in $cm^{-1}$ bei:
1655 [ C=O]; 2950 und 3450 [—OH].

Beispiel 11

150 g 4'-Chlorphenyl-1-chlorisopropylketon, 93 g 50%ige wäßrige Kaliumhydroxidlösung und 70 g Wasser werden unter Rühren auf 60°C aufgeheizt. Nach dem Zulauf von 0,23 g Tetrabutylammoniumhydrogensulfat, gelöst in 4,1 g Wasser (exotherme Reaktion), wird 30 Minuten bei 65—70°C ausgerührt und dann nochmals 0,93 g Tetrabutylammoniumhydrogensulfat, gelöst in 16,5 g Wasser, zugegeben. Das Gemisch wird eine Stunde bei 70—80°C ausgerührt. Nach dem Abtrennen der unteren wäßrigen Phase bei 60°C werden nochmals 70 g Wasser zugegeben, das Gemisch während 10 Minuten verrührt und durch Zugabe von ca. 3 g 80%iger Essigsäure ein pH-Wert von ca. 7 eingestellt. Die obere Wasserphase wird abgetrennt und das Produkt nochmals bei 60—70°C mit 70 g Wasser gewaschen. Die organische Phase wird mit 5,1 g Aktivkohle und 52 g Heptan versetzt und während 10 Minuten bei 50—55°C verrührt. Die Suspension wird über Hyflo klärfiltriert und das Heptan bei 80°C/2,7 · $10^3$ Pa abdestilliert. Es verbleiben 128,6 g 4'-Chlorphenyl-1-hydroxyisopropylketon, entsprechend 93,7% der Theorie.
Siedepunkt: 147°C/2,2 · $10^3$ Pa.
IR-Spektrum in 2%iger $CCl_4$-Lösung; Absorptionsbanden in $cm^{-1}$ bei:
1660 [ C=O]; 2950 und 3450 [—OH].

**Patentansprüche**

1. Verfahren zur Herstellung von Verbindungen der Formel I

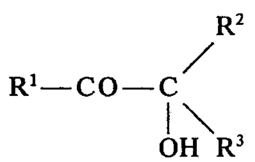

(I)

in der $R^1$ einen Phenylrest, welcher durch geradkettige oder verzweigte $C_1$—$C_4$-Alkylreste substituiert sein kann, bedeutet, $R^2$ und $R^3$ gleich oder verschieden sind, und einen geradkettigen oder verzweigten $C_1$—$C_5$-Alkylrest, einen $C_5$—$C_8$-Cycloalkylrest, welcher durch 1 bis 3 geradkettige oder verzweigte $C_1$—$C_5$-Alkylreste substituiert sein kann, oder zusammen mit dem C-Atom, an das sie gebunden sind, einen $C_5$—$C_8$-Cycloalkylrest, welcher durch 1 bis 3 geradkettige oder verzweigte $C_1$—$C_4$-Alkylreste substituiert sein kann, bedeuten, durch Umsetzung einer Verbindung der Formel II

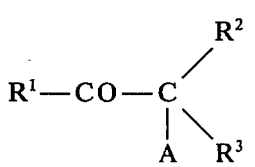

(II)

in der $R^1$, $R^2$ und $R^3$ wie oben definiert sind und A Chlor oder Brom bedeutet, mit Hydroxidionen erzeugenden Verbindungen in Gegenwart von Wasser oder in Gegenwart von Wasser und einem inerten organischen Lösungsmittel nach dem Phasentransfer Katalyse Verfahren, wobei ein Phasentransfer-Katalysator eingesetzt wird und die Verbindung der Formel II in Lösung oder geschmolzen

vorliegt.

2. Verfahren nach Anspruch 1, dadurch gekennzeichnet, daß A in Formel II Cl bedeutet.

3. Verfahren nach Anspruch 1, dadurch gekennzeichnet, daß man Verbindungen der Formel I herstellt, in der $R^1$ einen unsubstituierten oder durch eine oder mehrere $C_1-C_4$-Alkylgruppen substituierten Phenylrest, $R^2$ und $R^3$ beide Methyl oder zusammen mit dem C-Atom, an das sie gebunden sind, Cyclohexyl bedeuten, indem man entsprechende Verbindungen der Formel II mit Hydroxidionen erzeugenden Verbindungen umsetzt.

4. Verfahren nach Anspruch 1, dadurch gekennzeichnet, daß man als Hydroxidionen erzeugende Verbindungen Alkali- oder Erdalkalihydroxid einsetzt.

5. Verfahren nach Anspruch 1, dadurch gekennzeichnet, daß man als Hydroxidionen erzeugende Verbindungen KOH oder NaOH einsetzt.

6. Verfahren nach Anspruch 5, dadurch gekennzeichnet, daß die Verbindungen KOH und NaOH als wäßrige Lösungen eingesetzt werden.

7. Verfahren nach Anspruch 1, dadurch gekennzeichnet, daß die Verbindung der Formel II geschmolzen in dem Reaktionsgemisch vorliegt, und in Abwesenheit von inerten organischen Lösungsmitteln umgesetzt wird.

8. Verfahren nach Anspruch 1, dadurch gekennzeichnet, daß man als Phasentransfer-Katalysator eine Verbindung der Formel III

$$(R^a)\,(R^b)\,(R^c)\,(R^d)\,N^{\oplus}X^{\ominus} \qquad (III)$$

in der $R^a$, $R^b$, $R^c$ und $R^d$ gleich oder verschieden sind und geradkettige oder verzweigte Alkylreste, wobei alle 4 Alkylreste zusammen 4 bis 20 C-Atome besitzen, oder Benzyl bedeuten, und $X^{\ominus}$ $-HSO_4^{\ominus}$ oder $-Cl^{\ominus}$ darstellen, einsetzt.

9. Verfahren nach Anspruch 8, dadurch gekennzeichnet, daß man als Phasentransfer-Katalysator eine Verbindung aus der Reihe

$$(n-C_4H_9)_4\,N\,Cl,\ (n-C_4H_9)\,(C_2H_5)_3NHSO_4 \text{ und } (n-C_4H_9)_4NHSO_4$$

einsetzt.

10. Verfahren nach Anspruch 8, dadurch gekennzeichnet, daß man als Phasentransfer-Katalysator eine an ein polymeres Festharz gebundene Verbindung der Formel III verwendet.

11. Verfahren nach Anspruch 1, dadurch gekennzeichnet, daß man eine Verbindung aus der Reihe der Formeln

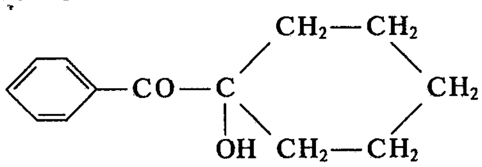

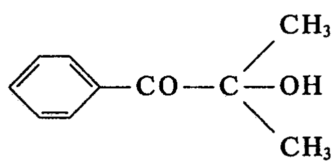

und

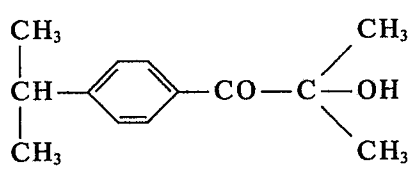

herstellt, indem man als entsprechende Verbindung der Formel II 1-Chlorcyclohexyl-phenylketon, 1-Chlorisopropyl-phenylketon oder 1-Chlorisopropyl-4'-isopropylphenylketon einsetzt.

## Claims

1. A process for the preparation of a compound of the formula I

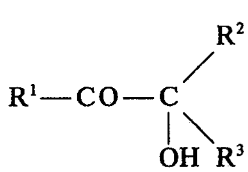

(I)

in which $R^1$ is a phenyl group which can be substituted by straight chain or branched $C_1-C_4$-alkyl groups, $R^2$ and $R^3$ are identical or different and are each a straight chain or branched $C_1-C_5$-alkyl

group, or a $C_5-C_8$-cycloalkyl group which can be substituted by 1 to 3 straight chain or branched $C_1-C_5$-alkyl groups, or together with the C atom to which they are bound they form a $C_5-C_8$-cycloalkyl group which can be substituted by 1 to 3 straight chain or branched $C_1-C_4$-alkyl groups, which process comprises reacting a compound of the formula II

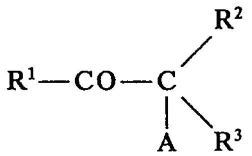

(II)

in which $R^1$, $R^2$ and $R^3$ are as defined above, and A is chlorine or bromine, with a compound releasing hydroxide ions, in the presence of water or in the presence of water and an inert organic solvent, by the phase-transfer catalysis method using a phase-transfer catalyst, the compound of the formula II being present in solution or in the molten form.

2. A process according to claim 1, wherein A in the formula II is chlorine.

3. A process according to claim 1, wherein a compound of the formula I is prepared, in which formula $R^1$ is a phenyl group which is unsubstituted or substituted by one or more $C_1-C_4$-alkyl groups, $R^2$ and $R^3$ are each methyl, or together with the C atom to which they are bound are cyclohexyl, which process comprises reacting a corresponding compound of the formula II with a compound releasing hydroxide ions.

4. A process according to claim 1, wherein the compound releasing hydroxide ions is an alkali metal hydroxide or alkaline-earth metal hydroxide.

5. A process according to claim 1, wherein the compound releasing hydroxide ions is KOH or NaOH.

6. A process according to claim 5, wherein the compound KOH or NaOH is used in the form of an aqueous solution.

7. A process according to claim 1, wherein the compound of the formula II is present in the melted form in the reaction mixture, and in the absence of inert organic solvents.

8. A process according to claim 1, wherein the phase-transfer catalyst is a compound of the formula III

$$(R^a)(R^b)(R^c)(R^d)N^{\oplus}X^{\ominus} \quad \text{(III)},$$

in which $R^a$, $R^b$, $R^c$ and $R^d$ are identical or different and are straight chain or branched alkyl groups, with all 4 alkyl groups together containing 4 to 20 carbon atoms, or they are benzyl, and $X^{\ominus}$ is $-HSO_4^{\ominus}$ or $-Cl^{\ominus}$.

9. A process according to claim 8, wherein the phasetransfer catalyst is a compound selected from the group consisting of:

$$(n-C_4H_9)_4N\,Cl,\ (n-C_4H_9)(C_2H_5)_3NHSO_4 \text{ and } (n-C_4H_9)_4NHSO_4.$$

10. A process according to claim 8, wherein the phasetransfer catalyst is a compound of the formula III bound to a polymeric solid resin.

11. A process according to claim 1, wherein a compound selected from the formulae consisting of

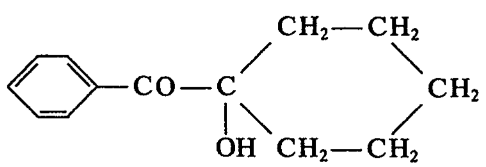

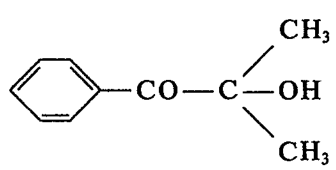

and

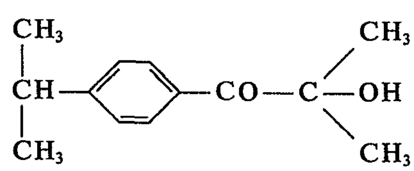

is prepared by using, as a corresponding compound of the formula II: 1-chlorocyclohexylphenyl ketone, 1-chloroisopropylphenyl ketone or 1-chloroisopropyl-4′-isopropylphenyl ketone.

**Revendications**

1. Procédé de préparation de composés répondant à la formule I:

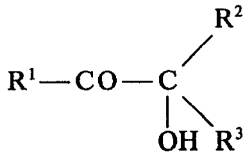

(I)

dans laquelle $R^1$ représente un radical phényle éventuellement porteur de radicaux alkyles, linéaires ou ramifiés, contenant de 1 à 4 atomes de carbone, et $R^2$ et $R^3$, qui peuvent être identiques l'un à l'autre ou différents l'un de l'autre, représentent chacun un radical alkyle en $C_1-C_5$ linéaire ou ramifié ou un radical cycloalkyle en $C_5-C_8$ éventuellement porteur d'un à trois radicaux alkyles en $C_1-C_5$ linéaires ou ramifiés, ou encore forment ensemble, et avec l'atome de carbone auquel ils sont unis, un radical cycloalkyle en $C_5-C_8$ qui peut porter de 1 à 3 radicaux alkyles en $C_1-C_4$ linéaires ou ramifiés par réaction d'un composé répondant à la formule II:

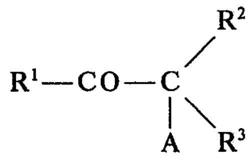

(II)

dans laquelle $R^1$, $R^2$ et $R^3$ ont les significations qui viennent d'être données et A représente le chlore ou le brome, par la méthode de catalyse avec transfer de phases, avec des composés engendrant des ions hydroxyles, en présence d'eau, ou en présence d'eau et d'un solvant organique inerte, procédé selon lequel on utilise un catalyseur de transfert de phases et on met en jeu le composé de formule II sous la forme d'une solution ou à l'état fondu.

2. Procédé selon la revendication 1 caractérisé en ce que A, dans la formule II, représente Cl.

3. Procédé selon la revendication 1 caractérisé en ce qu'on prépare des composés de formule I dans lesquels $R^1$ représente un radical phényle non substitué ou porteur d'un ou plusieurs radicaux alkyles en $C_1-C_4$, et $R^2$ et $R^3$ représentent chacun un radical méthyle ou forment ensemble, et avec l'atome de carbone qui les porte, un radical cyclohexyle, en faisant réagir des composés correspondants de formule II avec des composés engendrant des ions hydroxyles.

4. Procédé selon la revendication 1 caractérisé en ce qu'on utilise un hydroxyde de métal alcalin ou de métal alcalinoterreux comme composé engendrant des ions hydroxyles.

5. Procédé selon la revendication 1 caractérisé en ce qu'on utilise KOH ou NaOH comme composé engendrant des ions hydroxyles.

6. Procédé selon la revendication 5 caractérisé en ce que les composés KOH et NaOH sont mis en jeu sous la forme de solutions aqueuses.

7. Procédé selon la revendication 1 caractérisé en ce que le composé de formule II se trouve à l'état fondu dans le mélange réactionnel et est mis à réagir en l'absence de solvants organiques inertes.

8. Procédé selon la revendication 1 caractérisé en ce qu'on utilise, comme catalyseur de transfert de phases, un composé répondant à la formule III:

$$(R^a)(R^b)(R^c)(R^d)N^{\oplus}X^{\ominus} \qquad \text{(III)}$$

dans laquelle $R^a$, $R^b$, $R^c$ et $R^d$ représentent chacun, indépendamment les uns des autres, un radical alkyle linéaire ou ramifié, les quatre radicaux alkyles contenant ensemble de 4 à 20 atomes de carbone, ou un radical benzyle, et $X^{\ominus}$ représente $-HSO_4^{\ominus}$ ou $-Cl^{\ominus}$.

9. Procédé selon la revendication 8 caractérisé en ce qu'on utilise, comme catalyseur de transfert de phases, un composé pris dans l'ensemble constitué par $(n-C_4H_9)_4\,NCl$, $(n-C_4H_9)\,(C_2H_5)_3NHSO_4$ et $(n\text{-}C_4H_9)_4\,NHSO_4$.

10. Procédé selon la revendication 8 caractérisé en ce qu'on utilise, comme catalyseur de transfert de phase, un composé de formule III fixé sur une résine polymère solide.

11. Procédé selon la revendication 1 caractérisé en ce qu'on prépare un composé répondant à l'une des formules:

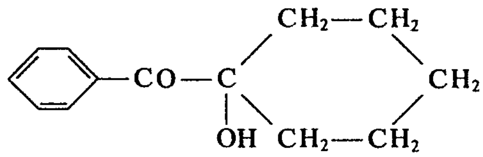

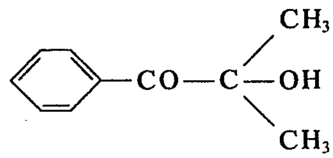

et

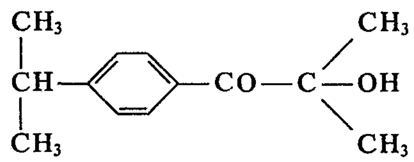

en utilisant, comme composé correspondant de formule II, la (chloro-1 cyclohexyl)-phényl-cétone, la (chloro-1 isopropyl)-phénylcétone ou la (chloro-1 isopropyl)-(isopropyl-4 phényl)-cétone.